# EUROPÄISCHE PATENTANMELDUNG

(11) **EP 3 605 132 A1**
(43) Veröffentlichungstag der Anmeldung: **05.02.2020**
(21) Anmeldenummer: 18186548.6
(22) Anmeldetag: 31.07.2018
(51) Int. Cl.: G01R 33/385, G01R 33/563

(54) **MAGNETRESONANZEINRICHTUNG UND VERFAHREN ZUR HERSTELLUNG EINER DIFFUSIONSGRADIENTENSPULE**

(71) Anmelder: Siemens Healthcare GmbH, 91052 Erlangen (DE)
(72) Erfinder: Dietz, Peter, 90762 Fürth (DE)

(57) **Zusammenfassung**

Magnetresonanzeinrichtung, umfassend eine Patientenaufnahme (3), wenigstens eine Diffusionsgradientenspule (10), wenigstens einen Magneten zur Erzeugung eines Grundmagnetfeldes und mehrere Gradientenspulen (4, 5, 6) zur Erzeugung von das Grundmagnetfeld überlagernden Gradientenfeldern, wobei sich das Grundmagnetfeld im Wesentlichen in der Patientenaufnahme (3) entlang einer ersten Richtung erstreckt und ein erster Gradient eines erstes Gradientenfeldes in der ersten Richtung sowie wenigstens ein weiterer Gradient eines weiteres Gradientenfeldes in einer weiteren Richtung orthogonal zu der ersten Richtung verläuft, wobei die Diffusionsgradientenspule (10) wenigstens eine in einer Ebene verlaufende Leiterschleife (11, 15) oder mehrere jeweils in parallelen Ebenen verlaufende Leiterschleifen (11, 15) aufweist, wobei die Diffusionsgradientenspule (10) derart in der Patientenaufnahme (3) in einer Einbauposition angeordnet ist, dass die Ebene oder die Ebenen der Leiterschleife (11, 15) orthogonal zu dem ersten Gradienten stehen und die Leiterschleife (11, 15) oder die Leiterschleifen (11, 15) in ihren jeweiligen Ebenen in Bezug zu dem wenigstens einen weiteren Gradientenfeld derart verlaufen, dass sich ein durch eine zeitliche Änderung des wenigstens einen weiteren Gradientenfeldes induzierter Strom in der Diffusionsgradientenspule (10) und/oder ein durch das Grundmagnetfeld erzeugtes, auf die Diffusionsgradientenspule (10) wirkendes Drehmoment aufhebt oder im Wesentlichen aufhebt.

## Beschreibung

Die Erfindung betrifft eine Magnetresonanzeinrichtung, umfassend eine Patientenaufnahme, wenigstens eine Diffusionsgradientenspule, wenigstens einen Magneten zur Erzeugung eines Grundmagnetfeldes und mehrere Gradientenspulen zur Erzeugung von das Grundmagnetfeld überlagernden Gradientenfeldern, wobei sich das Grundmagnetfeld im Wesentlichen in der Patientenaufnahme entlang einer ersten Richtung erstreckt und ein erster Gradient eines ersten Gradientenfeldes in der ersten Richtung sowie wenigstens ein weiterer Gradient eines weiteren Gradientenfeldes in einer weiteren Richtung orthogonal zu der ersten Richtung verläuft, wobei die Diffusionsgradientenspule wenigstens eine in einer Ebene verlaufende Leiterschleife oder mehrere jeweils in parallelen Ebenen verlaufende Leiterschleifen aufweist. Weiterhin betrifft die Erfindung ein Verfahren zur Herstellung einer Diffusionsgradientenspule.

Eine Diffusionsgradientenspule dient bei der Bildgebung mit einer Magnetresonanzeinrichtung dazu, zusätzlich zu den von den Gradientenspulen erzeugten Gradientenfeldern lokal durch einen Diffusionspuls eine große Magnetfeldstärke zu erzeugen, welche anstatt oder zusätzlich zu einem durch die Gradientenspulen erzeugten Diffusionsgradienten erzeugt wird. Die Diffusionsgradientenspule wird dazu in der Patientenaufnahme positioniert und kann insbesondere einen Körperteil eines Patienten, beispielsweise den Kopf, zumindest teilweise umschließen. Aufgrund der vergleichsweise geringen Abmessungen der Diffusionsgradientenspule wird die Ortsverteilung des Feldes der Diffusionsgradientenspule keinen linearen Gradienten erzeugen, sondern lokal stark variieren.

Hinsichtlich der Wechselwirkungen mit den weiteren Komponenten der Magnetresonanzeinrichtung werden an eine Diffusionsgradientenspule ähnliche Anforderungen gestellt wie an die Gradientenspulen der Magnetresonanzeinrichtung. Aufgrund der Anordnung der Diffusionsgradientenspule in der Magnetresonanzeinrichtung können bei ihrem Einsatz jedoch weitere Probleme bei auftreten.

Durch das Zusammenspiel der Ströme in der Diffusionsgradientenspule mit dem Grundmagnetfeld der Magnetresonanzeinrichtung können auf die Diffusionsgradientenspule wirkende Drehmomente entstehen. Die aus diesen Drehmomenten resultierenden Bewegungen der Diffusionsgradientenspule können für den Patienten als unangenehm empfunden werden. Finden aufgrund der Drehmomente starke Drehbewegungen der Diffusionsgradientenspule statt, so können in Teilen der Magnetresonanzeinrichtung vibrationsinduzierte Wirbelströme gebildet werden, welche die Bildgebung durch die Magnetresonanzeinrichtung stören können.

Weiterhin ist die Diffusionsgradientenspule bei ihrem Einsatz den Feldern der Gradientenspulen ausgesetzt, wobei durch die zeitliche Variation der Gradientenfelder und/oder des von der Diffusionsgradientenspule erzeugten Diffusionsgradientenfeldes wechselseitig Spannungen in den Spulen induziert werden können. Ab einer gewissen Höhe dieser induzierten Spannungen kann es zu Fehlern im Betrieb, beispielsweise dem Auftreten von Spannungsspikes, oder gar zum Ausfall von mit den jeweiligen Spulen verbundenen Verstärkern kommen. Kommt es durch die eingekoppelten Spannungen auch zu einem Stromfluss, so können insbesondere in der Diffusionsgradientenspule induzierte Wirbelströme zu einer Erwärmung der Diffusionsgradientenspule sowie zu Feldstörungen führen. Besonders bei einer im oder nahe am Field of View der Magnetresonanzeinrichtung platzierten Diffusionsgradientenspule können bereits kleine Wirbelströme zu Bildqualitätsstörungen führen.

Der Erfindung liegt daher die Aufgabe zugrunde, eine Magnetresonanzeinrichtung mit einer Diffusionsgradientenspule anzugeben, welche bei Betrieb der Diffusionsgradientenspule auftretende Störungen reduziert oder vermeidet.

Zur Lösung dieser Aufgabe ist erfindungsgemäß vorgesehen, dass die Diffusionsgradientenspule derart in der Patientenaufnahme in einer Einbauposition angeordnet ist, dass die Ebene oder die Ebenen der Leiterschleifen orthogonal zu dem ersten Gradienten stehen und die Leiterschleife oder die Leiterschleifen in ihren jeweiligen Ebenen in Bezug zu dem wenigstens einen weiteren Gradientenfeld derart verlaufen, dass sich ein durch eine zeitliche Änderung des wenigstens einen weiteren Gradientenfeldes induzierter Strom in der Diffusionsgradientenspule und/oder ein durch das Grundmagnetfeld erzeugtes, auf die Diffusionsgradientenspule wirkendes Drehmoment aufhebt oder im Wesentlichen aufhebt.

Die Magnetresonanzeinrichtung kann insbesondere eine zylindrische Patientenaufnahme umfassen, entlang deren Längsachse, welche im Folgenden als Z-Achse bezeichnet wird, das von dem Magneten der Magnetresonanzeinrichtung erzeugte Grundmagnetfeld verläuft. Zur Ortskodierung umfasst eine Magnetresonanzeinrichtung üblicherweise wenigstens drei Gradientenspulen, wobei durch diese ein erstes Gradientenfeld mit einem sich in der ersten Richtung des Grundmagnetfeldes, also in Z-Richtung, erstreckendem Gradienten sowie zwei weitere Gradientenfelder, deren weitere Gradienten sowohl orthogonal zu der ersten Richtung als auch orthogonal zueinander stehen, erzeugt werden. Die Richtungen der weiteren Gradienten werden dabei im Folgenden als X-Richtung und als Y-Richtung bezeichnet.

Die Diffusionsgradientenspule kann sich aus einer oder mehrerer Leiterschleifen zusammensetzen. Die Leiterschleifen können dabei zu einer oder mehreren Spuleneinheiten der Diffusionsgradientenspule miteinander verbunden sein. Durch unterschiedliche Zusammensetzung derartiger Leiterschleifen können verschiedene, von der Diffusionsgradientenspule zu erzeugende Feldprofile, wie beispielsweise fokussierende Felder, erzeugt werden.

Der Vorteil der erfindungsgemäßen Lösung besteht darin, dass aufgrund der Anordnung der Diffusionsgradientenspule in der Patientenaufnahme unerwünschte Einflüsse und Störungen beim Betrieb der Diffusionsgradientenspule bzw. der Magnetresonanzeinrichtung verringert und/oder verhindert werden können. Dazu ist die Diffusionsgradientenspule in der Einbauposition positioniert, in der die Ebene oder die Ebenen der Leiterschleifen der Diffusionsgradientenspule orthogonal zu dem ersten Gradienten stehen. Dies bedeutet, dass sich eine Längsachse der Diffusionsgradientenspule in Z-Richtung erstreckt, und dass die Leiterschleife oder die Leiterschleifen der Diffusionsgradientenspule orthogonal dazu, also jeweils in einer XY-Ebene, verlaufen. Weiterhin ist in der Einbauposition vorgesehen, dass die Leiterschleifen derart in ihren jeweiligen Ebenen in Bezug zu dem wenigstens einen weiteren Gradientenfeld verlaufen, dass sich durch wenigstens eines der weiteren Gradientenfelder oder durch eine zeitliche Änderung wenigstens eines der weiteren Gradientenfelder erzeugte Einflüsse aufheben oder im Wesentlichen aufheben. Unter Berücksichtigung des Verlaufs des wenigstens einen weiteren Gradientenfeldes sowie der Anzahl und der Form der Leiterschleifen der Diffusionsgradientenspule kann in der Einbauposition somit eine symmetrische oder im Wesentlichen symmetrische Durchdringung der Diffusionsgradientenspule durch das wenigstens eine weitere Gradientenfeld erreicht werden, so dass sich ein durch eine zeitliche Änderung des weiteren Gradientenfeldes induzierter Strom in der Diffusionsgradientenspule und/oder ein durch das Grundmagnetfeld erzeugtes, auf die Diffusionsgradientenspule wirkendes Drehmoment aufhebt oder im Wesentlichen aufhebt.

Im Wesentlichen aufheben ist in diesem Zusammenhang derart zu verstehen, dass z. B. die Summe aus zwei Komponenten eines Stromes oder eines Drehmoments, welche auf die Diffusionsgradientenspule wirken oder in dieser fließen, unterschiedliche Vorzeichen aufweisen, so dass die Summe beispielsweise nur 20% oder 10% des Betrages einer der Komponenten beträgt. Das auf die Diffusionsgradientenspule wirkende Drehmoment bzw. der in der Diffusionsgradientenspule fließende Strom kann somit ganz oder teilweise kompensiert werden, so dass auch die Auswirkungen auf die Diffusionsgradientenspule, wie Bewegungen, Vibrationen und/oder Erwärmung, und somit auch Auswirkungen auf einen Patienten oder die Gradientenspulen vorteilhaft reduziert werden.

Eine zeitliche Änderung des wenigstens einen Gradientenfeldes kann beispielsweise bei dem Auslesen von Bilddaten aufgrund der gemeinhin bei Magnetresonanzeinrichtungen eingesetzten Ortskodierung und/oder bei Einsatz eines bei der Diffusionsbildgebung üblicherweise eingesetzten EPI-Readout-Gradienten auftreten, wobei durch die sich zeitlich verändernde magnetische Feldstärke bzw. Flussdichte des wenigstens einen weiteren Gradientenfeldes in der Diffusionsgradientenspule ein Strom induziert werden kann.

Ein Drehmoment kann in der Diffusionsgradientenspule auftreten, wenn in dieser, beispielsweise zur Erzeugung des Diffusionsgradientenfeldes, ein Strom fließt. Aufgrund der orthogonalen Ausrichtung der Leiterschleife oder der Leiterschleifen der Diffusionsgradientenspule zu dem Grundmagnetfeld entstehen in diesem Fall aufgrund des Stromflusses Lorentzkräfte, welche in der Ebene der Leiterschleife oder der Leiterschleifen der Diffusionsgradientenspule wirken. In der sich in einer Ebene erstreckenden Leiterschleife gibt es dabei aufgrund ihrer Schleifenform einen räumlich getrennten Strom in eine Richtung und einen räumlich getrennten Strom in die Gegenrichtung. So entstehen durch das Grundmagnetfeld auf die Leiterschleifen wirkende Lorentzkräfte in entgegengesetzten Richtungen in der Ebene der Leiterschleife bzw. Leiterschleifen, welche kein oder im Wesentlichen kein auf die Diffusionsgradientenspule wirkendes Drehmoment erzeugen.

Das wenigstens eine weitere Gradientenfeld kann in einer in der XY-Ebene liegenden Leiterschleife nur durch seine sich in Z-Richtung erstreckende Komponente B_{Z} einen Strom induzieren, da nur B_{Z} durch die Fläche der Leiterschleife dringen kann. Die intrinsische Eigenschaft eines Gradientenfeldes ist es, dass B_{Z} die Form eines Gradienten hat, d.h. dass die Komponente B_{Z,X} eines Gradientenfeldes mit einem sich in X-Richtung erstreckenden Gradienten beispielsweise an Positionen +X₀ und -X₀ eine gleiche Amplitude, jedoch ein unterschiedliches Vorzeichen hat. Ein durch eine zeitliche Änderung eines weiteren Gradientenfeldes mit einem sich in X-Richtung erstreckenden Gradienten induzierter Strom kann sich somit bei einer entsprechenden Anordnung der Leiterschleife in Bezug zu dem Gradientenfeld kompensieren. Entsprechend gilt dies selbstverständlich bei einem weiteren Gradientenfeldes mit einem sich in Y-Richtung erstreckenden Gradienten für die Komponente B_{Z,Y} an den Positionen +Y₀ und -Y₀.

Durch die erfindungsgemäße Anordnung einer Diffusionsgradientenspule werden vorteilhaft auf die Diffusionsgradientenspule wirkende Drehmomente und Einkopplungen in Form von induzierten Strömen bzw. Spannungen vermieden. Vorteilhaft können somit Unannehmlichkeiten oder gar eine Gefährdung des Patienten sowie eine Schädigung der Hardware, insbesondere mit der Diffusionsgradientenspule und/oder den Gradientenspulen der Magnetresonanzeinrichtung verbundene Verstärker, vermieden werden.

Eine Diffusionsgradientenspule, deren Längsachse parallel zur Richtung des Grundmagnetfeldes bzw. deren Leiterschleife oder Leiterschleifen orthogonal zur Richtung des Grundmagnetfelds positioniert sind, ist darüber hinaus auch besonders effektiv, da die komplette Leiterlänge der Leiterschleife bzw. der Leiterschleifen der Diffusionsgradientenspule vor allem eine Feldkomponente in der Richtung des Grundmagnetfeldes, also ein B_{Z}-Feld bzw. ein, für eine Bildgebung mit der Magnetresonanzeinrichtung vorteilhaft einsetzbares Nutzfeld erzeugt. Diese sind, ähnlich wie ein sich Z-Richtung erstreckender Standardgradient, effizienter als transversale, das heißt in X- oder in Y-Richtung verlaufende, Gradienten. Dadurch werden vorteilhaft die größten Feldamplituden des von der Diffusionsgradientenspule erzeugten Feldes auf das Zielobjekt gerichtet, wo sie für die Bildgebung ohnehin benötigt werden. Große Feldkomponenten in einer Richtung orthogonal zur Z-Richtung können somit vermieden werden, wodurch unerwünschte Wechselwirkungen mit dem Patienten wie Peripheral Nerve Stimulation (PNS) in einer benachbarten Körperregion des Patienten vermieden werden können. Die Anordnungen der Leiterschleife oder der Leiterschleifen in der XY-Ebene ist weiterhin für gängige, unter Einsatz einer Diffusionsgradientenspule durchgeführte Untersuchungen, wie beispielsweise an einem Kopf oder einer Prostata des Patienten, gut möglich, da der Patient dazu in der Regel entlang der Z-Richtung in der Magnetresonanzeinrichtung positioniert wird.

Erfindungsgemäß kann vorgesehen sein, dass die Diffusionsgradientenspule derart zu dem ersten Gradientenfeld steht, dass sich ein durch das erste Gradientenfeld erzeugtes, auf die Diffusionsgradientenspule wirkendes Drehmoment und/oder ein durch eine zeitliche Änderung des ersten Gradientenfeldes in der Diffusionsgradientenspule induzierter Strom aufhebt oder im Wesentlichen aufhebt. Das heißt, dass nicht nur die Positionierung der Diffusionsgradientenspule orthogonal zu der Z-Richtung derart erfolgt, dass durch eines oder mehrere weitere Gradientenfelder keine oder im Wesentlichen keine Störungen auftreten, sondern dass die Diffusionsgradientenspule zusätzlich auch in der ersten Richtung, also der Z-Richtung, derart in der Einbauposition angeordnet ist, dass sich ein durch das erste Gradientenfeld erzeugtes, auf die Diffusionsgradientenspule wirkendes Drehmoment und/oder ein durch eine zeitliche Änderung des ersten Gradientenfeldes in der Diffusionsgradientenspule induzierter Strom aufhebt oder im Wesentlichen aufhebt.

Bevorzugt erfolgt die Anordnung der Diffusionsgradientenspule in der Einbauposition derart, dass sich sowohl ein durch das Grundmagnetfeld erzeugtes Drehmoment als auch ein durch eine zeitliche Änderung des ersten Gradientenfeldes erzeugter Strom aufhebt oder im Wesentlichen aufhebt und dass für wenigstens zwei weitere Gradientenfelder, deren Gradienten orthogonal zueinander und orthogonal zu dem Gradienten des ersten Gradientenfeld stehen, eine Kompensation von induzierten Strömen wie vorangehend beschrieben erfolgt. Somit können alle auf die Diffusionsgradientenspule wirkenden Drehmomente bzw. alle in der Diffusionsgradientenspule induzierten Ströme vorteilhaft reduziert oder kompensiert werden, so dass insgesamt keine oder nur geringe Drehmomente auf die Diffusionsgradientenspule wirken bzw. keine oder nur geringe Ströme in der Diffusionsgradientenspule induziert werden.

In einer bevorzugten Ausgestaltung der Erfindung kann vorgesehen sein, dass eine Leiterschleife der Diffusionsgradientenspule und/oder alle Leiterschleifen der Diffusionsgradientenspule symmetrisch um ein Isozentrum der Gradientenfelder angeordnet sind. Unter dem Isozentrum ist hierbei der Punkt in der Patientenaufnahme zu verstehen, an dem eine Vorzeichenumkehr der B_{Z}-Komponente des ersten Gradientenfeldes und des wenigstens einen weiteren Gradientenfeldes stattfindet. Das heißt, dass im Isozentrum die B_{Z}-Komponente des ersten und des wenigstens einen weiteren Gradientenfeldes jeweils Null ist. Bei einem entlang der ersten Richtung symmetrisch verlaufenden ersten Gradientenfeld handelt es sich bei dem Isozentrum folglich um den Mittelpunkt des ersten Gradientenfeldes. In einer Leiterschleife, welche in der XY-Ebene durch das Isozentrum, also in der XY-Ebene durch den Punkt Z=0, angeordnet ist, findet keine Einkopplung durch das erste Gradientenfeld statt, da es dort keine B_{Z}-Komponente des ersten Gradientenfeldes gibt.

Eine Diffusionsgradientenspule, welche eine einzelne Leiterschleife umfasst, kann deshalb derart symmetrisch um das Isozentrum angeordnet werden, dass die eine Leiterschleife in der XY-Ebene durch das Isozentrum des ersten Gradientenfeldes angeordnet ist. Eine Diffusionsgradientenspule, welche mehr als eine Leiterschleife umfasst, kann dabei derart angeordnet werden, dass die Leiterschleifen symmetrisch um das Isozentrum herum angeordnet sind, so dass sich insgesamt der Einfluss durch das erste Gradientenfeld auf die Diffusionsgradientenspule kompensiert oder im Wesentlichen kompensiert. Bei einer in Reihe geschalteten Serie von Leiterschleifen kann somit folglich erreicht werden, dass sich alle positiven und alle negativen Einkopplungen in die Diffusionsgradientenspule in der Summe aufheben. Die Diffusionsgradientenspule kann insbesondere aus an verschiedenen Positionen in Z-Richtung jeweils in einer XY-Ebene verlaufenden Leiterschleifen bestehen, wobei ein globaler effektiver Mittelpunkt der Diffusionsgradientenspule, an dem eine Symmetrie hinsichtlich der B_{Z}-Komponenten des ersten und/oder des wenigstens einen weiteren Gradientenfeldes besteht, sich im Isozentrum des ersten Gradientenfeldes befindet, so dass sich auftretende Drehmomente und/oder induzierte Ströme zumindest im Wesentlichen kompensieren.

Erfindungsgemäß kann vorgesehen sein, dass die Diffusionsgradientenspule mehrere Leiterschleifen aufweist, wobei die Leiterschleifen konzentrisch und/oder jeweils entlang der Längsachse der Diffusionsgradientenspule versetzt angeordnet sind und/oder jeweils unterschiedliche Durchmesser aufweisen. Dabei ist es besonders bevorzugt, wenn die Leiterschleifen der Diffusionsgradientenspule symmetrisch zu einem Mittelpunkt der Diffusionsgradientenspule sind, da dadurch die Anordnung der Diffusionsgradientenspule in der Einbauposition insbesondere im Fall von symmetrischen Gradientenfeldern vereinfacht wird.

Für die Leiterschleife oder die Leiterschleifen kann erfindungsgemäß vorgesehen sein, dass sie in ihrer Ebene einen runden, ovalen, rechteckigen oder polygonförmigen Querschnitt aufweisen. Auch bezüglich der Form der Leiterschleifen kann vorgesehen sein, dass diese eine Symmetrie bezüglich einer Mittelachse der Diffusionsgradientenspule aufweisen, wenn die Diffusionsgradientenspule in einer Magnetresonanzeinrichtung mit einem symmetrischen ersten Gradientenfeld und/oder symmetrischen weiteren Gradientenfeldern eingesetzt werden soll. Bei bereichsweise asymmetrischen Gradientenfeldern ist es möglich, den Einfluss einer Asymmetrie eines oder mehrerer der Gradientenfelder durch entsprechende Unterschiede in dem Verlauf und/oder in der Querschnittsform einer oder mehrerer Leiterschleifen der Diffusionsgradientenspule zu kompensieren, so dass auch bei einem bereichsweise asymmetrischen ersten und/oder asymmetrischen weiteren Gradientenfeldern eine Anordnung der Diffusionsgradientenspule in der erfindungsgemäß vorgesehenen Einbauposition aufgrund des auf die Asymmetrie angepassten Verlaufs einer oder mehrerer Leiterschleifen möglich ist.

In einer bevorzugten Ausgestaltung der Erfindung kann vorgesehen sein, dass die Diffusionsgradientenspule für eine Messung am Kopf und/oder einer Prostata eines in der Patientenaufnahme positionierten Patienten ausgebildet ist. Die Diffusionsgradientenspule weist dazu eine Ausdehnung in Längsrichtung sowie einen Durchmesser der Leiterschleifen auf, welche die Anordnung der Diffusionsgradientenspule am Kopf und/oder im Bereich einer Prostata des Patienten ermöglicht. Für eine Messung an der Prostata eines Patienten kann die Diffusionsgradientenspule beispielsweise zwischen den Oberschenkeln eines Patienten positioniert werden. Für eine Messung am Kopf des Patienten kann vorgesehen sein, dass die Diffusionsgradientenspule ganz oder bereichsweise um den Kopf des Patienten herum positioniert wird.

Erfindungsgemäß kann vorgesehen sein, dass die Magnetresonanzeinrichtung wenigstens eine in der Patientenaufnahme und/oder in einer in der Patientenaufnahme angeordneten oder anordenbaren Patientenlagerungseinrichtung ausgebildete Halterung zur entnehmbaren und spielfreien Aufnahme der Diffusionsgradientenspule in der Einbauposition aufweist. Die Ausbildung des ersten Gradientenfeldes und des wenigstens eines weiteren Gradientenfeldes der Magnetresonanzeinrichtung ergibt sich aus den Gradientenspulen der Magnetresonanzeinrichtung. Es kann dabei für ein oder mehrere, der Magnetresonanzeinrichtung zugeordnete Diffusionsgradientenspulen jeweils wenigstens eine in der Patientenaufnahme und/oder an der Patientenlagerungseinrichtung ausgebildete Halterung vorgesehen sein, in der die der Magnetresonanzeinrichtung zugeordneten Diffusionsgradientenspulen in ihrer jeweiligen Einbauposition angeordnet werden können.

Die Anordnung der Diffusionsgradientenspulen in der Halterung erfolgt dabei derart, dass die Diffusionsgradientenspulen aus der Halterung entnommen werden können, wenn sie nicht benötigt werden. Weiterhin erfolgt die Aufnahme der Diffusionsgradientenspulen in der Halterung spielfrei, so dass eine möglichst exakte Positionierung der Diffusionsgradientenspule in der Einbauposition ermöglicht wird. Beispielsweise ist es möglich, dass eine Halterung für eine zur Aufnahme einer zur Messung am Kopf eines Patienten ausgebildeten Diffusionsgradientenspule und/oder eine weitere Halterung zur Aufnahme einer zur Messung an einer Prostata des Patienten ausgebildeten Diffusionsgradientenspule vorgesehen ist. Selbstverständlich ist es möglich, dass zu weiteren Verwendungszwecken auch noch weitere und/oder abweichende Halterungen für die Diffusionsgradientenspule in der Patientenaufnahme und/oder an der Patientenlagerungseinrichtung vorgesehen sind.

Für die Halterung kann erfindungsgemäß vorgesehen sein, dass sie zur formschlüssigen Aufnahme der Diffusionsgradientenspule ausgebildet ist und/oder dass die Halterung zu Befestigungsmitteln der Diffusionsgradientenspule korrespondierende Gegenstücke aufweist. Die Halterung kann beispielsweise als eine Mulde in einer Innenverkleidung der Patientenaufnahme und/oder in der Patientenlagerungseinrichtung ausgebildet sein, in die die Diffusionsgradientenspule eingesetzt werden kann. Dabei ist es nicht notwendig, dass die gesamte Diffusionsgradientenspule in der Mulde aufgenommen wird, es reicht vielmehr aus, wenn diese abschnittweise, beispielsweise mit einem Teil ihrer äußeren Begrenzung, formschlüssig in die Mulde eingreift, so dass eine entnehmbare und spielfreie Anordnung der Diffusionsgradientenspule in der Halterung gegeben ist. Zusätzlich oder alternativ dazu ist es möglich, dass die Halterung Gegenstücke zu Befestigungsmitteln, wie beispielsweise Schrauben oder Zapfen der Diffusionsgradientenspule aufweist, so dass ein Anschrauben und/oder Anstecken der Diffusionsgradientenspule an die Halterung ermöglicht wird.

Erfindungsgemäß kann vorgesehen sein, dass die Magnetresonanzeinrichtung eine Patientenlagerungseinrichtung umfasst, wobei sich die erste Richtung orthogonal oder parallel zu einer Patientenlagerungsfläche der Patientenlagerungseinrichtung erstreckt und die Diffusionsgradientenspule in oder an der Patientenlagerungseinrichtung angeordnet ist. Bei einer ersten Richtung bzw. Z-Richtung, welche sich parallel zu einer Patientenlagerungsfläche der Patientenlagerungseinrichtung erstreckt, mithin also in einer waagerechten Richtung insbesondere innerhalb einer Patientenaufnahme der Magnetresonanzeinrichtung verläuft, erfolgt die Anordnung einer Diffusionsgradientenspule derart, dass die Längsachse der Diffusionsgradientenspule ebenfalls parallel zu der ersten Richtung ist. Dies bedeutet, dass die Ebenen in der Leiterschleife orthogonal zu der Patientenlagerungsfläche der Patientenlagerungseinrichtung angeordnet sind.

Bei einer Open-MR-Einrichtung verläuft die erste Richtung, also die Richtung des Grundmagnetfeldes, in der Regel orthogonal zu der Patientenlagerungsfläche der Patientenlagerungseinrichtung, so dass die Anordnung einer Diffusionsgradientenspule derart erfolgt, dass die Ebenen der Leiterschleifen der Diffusionsgradientenspule parallel zu der Patientenlagerungsfläche der Patientenlagerungseinrichtung verlaufen, mithin also die Längsachse der Diffusionsgradientenspule orthogonal zu der Patientenlagerungsfläche der Patientenlagerungseinrichtung steht. Die Geometrie in einer Open-MR-Einrichtung unterscheidet sich verglichen mit einer konventionellen Magnetresonanzeinrichtung lediglich darin, dass die Anordnung der Diffusionsgradientenspule in Bezug zu der Patientenlagerungsfläche der Patientenlagerungseinrichtung bzw. zu dem Patienten anders ist.

Weiterhin kann erfindungsgemäß vorgesehen sein, dass wenigstens ein mit der Diffusionsgradientenspule verbundenes Anschlussmittel, insbesondere eine elektrische Leitung oder ein Kühlmittelschlauch, und/oder wenigstens ein der Diffusionsgradientenspule zugeordneter Sensor in der Patientenlagerungseinrichtung angeordnet ist. Dabei kann bevorzugt vorgesehen sein, dass das wenigstens eine Anschlussmittel im Bereich der Halterung für die Diffusionsgradientenspule angeordnet ist, so dass ein Anschluss der Diffusionsgradientenspule bei Einsetzen dieser in die Halterung erfolgt. Eine in der Patientenlagerungseinrichtung verlaufende elektrische Leitung kann beispielsweise zur Stromversorgung der Diffusionsgradientenspule dienen. Über einen Kühlmittelschlauch kann der Diffusionsgradientenspule beispielsweise ein Kühlmittel, insbesondere ein Öl für eine Ölkühlung, zugeführt werden. Eine Ölkühlung der Diffusionsgradientenspule bietet den Vorteil, dass in diesem Fall kein zusätzliches Wasser in den Aufnahmebereich der Magnetresonanzeinrichtung gebracht wird. Zusätzlich oder alternativ dazu kann auch wenigstens ein der Diffusionsgradientenspule zugeordneter Sensor in der Patientenlagerungseinrichtung angeordnet sein. Dieser kann beispielsweise der Strommessung und/oder einer Temperaturmessung und/oder der Messung eines durch die Diffusionsgradientenspule erzeugten Diffusionsgradientenfeldes dienen.

Für ein erfindungsgemäßes Verfahren zur Herstellung einer Diffusionsgradientenspule für eine erfindungsgemäße Magnetresonanzeinrichtung ist vorgesehen, dass der Verlauf und/oder die Querschnittsform der wenigstens einen Leiterschleife der Diffusionsgradientenspule und/oder die Anordnung mehrerer Leiterschleifen der Diffusionsgradientenspule zueinander in Abhängigkeit des von den Gradientenspulen der Magnetresonanzeinrichtung erzeugten, wenigstens einen weiteren Gradientenfeldes und der Position der Diffusionsgradientenspule in der Patientenaufnahme unter Verwendung eines Optimierungsalgorithmus bestimmt wird, wobei als Optimierungsbedingung des Optimierungsalgorithmus eine Minimierung eines durch eine zeitliche Änderung des wenigstens einen weiteren Gradientenfeldes induzierten Stromes in der Diffusionsgradientenspule verwendet wird.

Auf diese Weise wird es vorteilhaft ermöglicht, dass die Diffusionsgradientenspule durch die Optimierung des Verlaufs und/oder der Querschnittsform der wenigstens einen Leiterschleife der Diffusionsgradientenspule für den Einsatz einer Magnetresonanzeinrichtung optimiert wird, das heißt, dass bei einem Anordnen der Diffusionsgradientenspule in ihrer Einbauposition in der Magnetresonanzeinrichtung der Einfluss von in der Diffusionsgradientenspule induzierten Strömen durch das wenigstens eine weitere Gradientenfeld vorteilhaft verringert oder vermieden werden kann. Der Verlauf und/oder die Querschnittsform der wenigstens einen Leiterschleife wird mit Hilfe eines Optimierungsalgorithmus bestimmt, wobei die Optimierungsbedingung für den Algorithmus die Minimierung eines durch eine zeitliche Änderung des wenigstens einen weiteren Gradientenfeldes induzierten Stromes in der Diffusionsgradientenspule verwendet wird. Selbstverständlich ist es möglich, dass weitere Optimierungsbedingungen und/oder dass Randbedingungen für den Algorithmus festgelegt werden können. Als weitere Optimierungsbedingung kann selbstverständlich, insbesondere in Abhängigkeit des Verwendungszwecks der Diffusionsgradientenspule, eine von dem Diffusionsgradientenfeld zu erreichende maximale Feldstärke und/oder eine Gradientenstärke und/oder eine Geometrie des Diffusionsgradientenfeldes verwendet werden. Als Randbedingung kann beispielsweise eine Anzahl von Leiterschleifen der Diffusionsgradientenspule und/oder ein minimaler Durchmesser der einzelnen Leiterschleifen der Diffusionsgradientenspule festgelegt werden, wodurch sichergestellt wird, dass das Optimierungsergebnis auch für bestimmte Untersuchungszwecke eingesetzt werden kann. Selbstverständlich ist es möglich, dass die Optimierung auch für zwei weitere Gradientenfelder mit insbesondere orthogonal zueinander und orthogonal zu der Richtung des Grundmagnetfelds stehenden Gradienten durchgeführt wird.

Zusätzlich kann erfindungsgemäß vorgesehen sein, dass als zusätzliche Optimierungsbedingung eine Minimierung eines durch das erste Gradientenfeld erzeugten, auf die Diffusionsgradientenspule wirkenden Drehmoments und/oder eines durch eine zeitliche Änderung des ersten Gradientenfeldes in der Diffusionsgradientenspule induzierten Stroms verwendet wird. Auf diese Weise kann sichergestellt werden, dass auch durch ein erstes Gradientenfeld mit einem entlang der Richtung des Grundmagnetfelds verlaufenden ersten Gradienten keine oder nur geringe negative Einflüsse auf die Diffusionsgradientenspule und/oder auf mit Hilfe der Diffusionsgradientenspule durchgeführte Messungen erfolgt.

Weitere Vorteile und Einzelheiten der Erfindung ergeben sich aus den im Folgenden beschriebenen Ausführungsbeispielen sowie anhand der Zeichnungen. Dabei zeigen:
- Fig. 1: eine schematische Stirnansicht eines ersten Ausführungsbeispiels einer erfindungsgemäßen Magnetresonanzeinrichtung,
- Fig. 2: eine schematische Seitenansicht des ersten Ausführungsbeispiels einer erfindungsgemäßen Magnetresonanzeinrichtung,
- Fig. 3: eine schematische Seitenansicht eines zweiten Ausführungsbeispiels einer erfindungsgemäßen Magnetresonanzeinrichtung,
- Fig. 4: eine schematische Darstellung der Anordnung von Diffusionsgradientenspulen an einem Patienten,
- Fig. 5 - Fig. 7: verschiedene Ansichten einer Diffusionsgradientenspule, und
- Fig. 8-Fig. 10: schematische Darstellungen verschiedener Verläufe einer Leiterschleife einer Diffusionsgradientenspule.

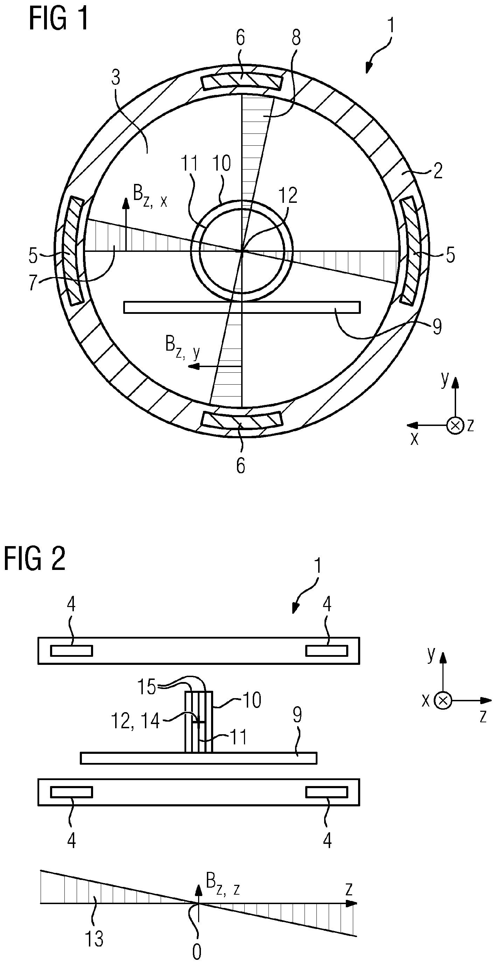

In Fig. 1 ist eine erfindungsgemäße Magnetresonanzeinrichtung 1 dargestellt. Diese umfasst ein zylinderförmiges Gehäuse 2, welches eine als Bore ausgeführte Patientenaufnahme 3 seitlich umschließt. Die Magnetresonanzeinrichtung 1 umfasst weiterhin einen in Fig. 1 nicht dargestellten Magneten zur Erzeugung eines Grundmagnetfeldes, welches sich in Fig. 1 in die Zeichenebene hinein, das heißt entlang der Z-Achse, erstreckt. Weiterhin umfasst die Magnetresonanzeinrichtung 1 mehrere Gradientenspulen 4, 5, 6, welche zur Erzeugung von das Gradientenfeld überlagernden Gradientenfeldern dienen.

Die Gradientenspulen 4 dienen der Erzeugung eines ersten Gradientenfeldes, dessen erster Gradient sich entlang des in Z-Richtung ausgedehnten Grundmagnetfeldes erstreckt. Die Gradientenspulen 4 sind in der einen Seitenansicht der erfindungsgemäßen Magnetresonanzeinrichtung 1 zeigenden Fig. 2 schematisch dargestellt. In Fig. 1 schematisch dargestellt sind die Gradientenspulen 5, welche der Erzeugung eines weiteren Gradientenfeldes mit einem hier in X-Richtung verlaufenden, weiteren Gradienten dienen. Durch die Gradientenspulen 6 wird entsprechend ein weiteres Gradientenfeld, dessen weiterer Gradient sich in Y-Richtung erstreckt, erzeugt. Die Anordnung sowie die Form der Gradientenspulen 4, 5, 6 ist in den Fig. 1 und 2 aus Gründen der Übersichtlichkeit rein beispielhaft.

Der Verlauf der Z-Komponente B_{Z,X} des weiteren Gradientenfeldes mit dem sich in X-Richtung erstreckenden weiteren Gradienten ist schematisch in dem Graphen 7 dargestellt, entsprechend zeigt der Graph 8 die Z-Komponente B_{Z,Y} des weiteren Gradientenfeldes mit dem sich in Y-Richtung erstreckenden weiteren Gradienten. Der sich in X-Richtung erstreckende weitere Gradient und der sich in Y-Richtung erstreckende weitere Gradient sind sowohl orthogonal zueinander als auch orthogonal zu dem sich in Z-Richtung erstreckenden ersten Gradienten des ersten Gradientenfeldes.

Im Inneren der Patientenaufnahme 3 sind weiterhin schematisch eine Patientenlagerungseinrichtung 9, welche beispielsweise als Liege ausgeführt sein kann, sowie eine Diffusionsgradientenspule 10 dargestellt. Die Diffusionsgradientenspule 10 umfasst eine oder mehrere Leiterschleifen 11, welche sich jeweils in einer XY-Ebene erstrecken. Bei mehreren Leiterschleifen 11 sind die jeweiligen XY-Ebenen, in denen sich die Leiterschleifen erstrecken, parallel und voneinander in Z-Richtung beabstandet. In diesem Ausführungsbeispiel weisen die Leiterschleifen 11 der Diffusionsgradientenspule 10 einen kreisförmigen Querschnitt sowie einen konstanten Durchmesser auf. Schematisch ist eine Leiterschleife 11 der Diffusionsgradientenspule 10 in Fig. 1 dargestellt.

Die Diffusionsgradientenspule 10 ist in der Patientenaufnahme 3 in einer Einbauposition derart angeordnet, dass die Leiterschleife 11 orthogonal zu dem sich in Z-Richtung erstreckenden ersten Gradienten bzw. dem Grundmagnetfeld steht. Weiterhin ist die Leiterschleife 11 in ihrer Ebene derart angeordnet, dass sie sowohl zu dem in X-Richtung verlaufenden weiteren Gradienten als auch zu dem in Y-Richtung verlaufenden weiteren Gradienten symmetrisch zu einer Symmetrieachse 12 der weiteren Gradientenfelder angeordnet ist, das heißt der Mittelpunkt der kreisförmigen Leiterschleife 11 liegt auf der Symmetrieachse 12. Die Symmetrieachse 12 beschreibt die Achse, bei denen die jeweils symmetrisch in X-Richtung bzw. in Y-Richtung verlaufenden weiteren Gradientenfelder keine Z-Komponente B_{Z,X} bzw. B_{Z,Y} aufweisen. Aufgrund der symmetrischen Ausgestaltung der weiteren Gradientenfelder in X- und in Y-Richtung fällt die Symmetrieachse 12 in der Darstellung in Fig. 1 mit der Mittelachse der Patientenaufnahme 3 bzw. der Magnetresonanzeinrichtung 1 zusammen. Bei einer Asymmetrie in einem oder beiden der weiteren Gradientenfelder kann die Symmetrieachse 12 eine Kurve sein und einen von einer Geraden und insbesondere einen von der Mittelachse der Patientenaufnahme 3 abweichenden Verlauf ausweisen.

Aufgrund der symmetrischen Anordnung der Leiterschleife 11 um die Symmetrieachse 12 und den symmetrischen Verlauf der Z-Komponenten B_{Z,X} bzw. B_{Z,Y} um die Symmetrieachse 12 wird ein durch eine zeitliche Änderung eines der weiteren Gradientenfelder durch die Z-Komponenten B_{Z,X} bzw. B_{Z,Y} in der Leiterschleife 11 der Diffusionsgradientenspule 10 induzierter Strom aufgehoben beziehungsweise im Wesentlichen aufgehoben.

In Fig. 2 ist eine schematische Seitenansicht der erfindungsgemäßen Magnetresonanzeinrichtung 1 dargestellt. In dem Graphen 13 ist der Verlauf der Z-Komponente B_{Z,Z} des ersten Gradientenfeldes, welches durch die Gradientenspulen 4 erzeugt wird, schematisch dargestellt. Die Diffusionsgradientenspule 10 ist symmetrisch um ein Isozentrum 14 des ersten Gradientenfeldes mit dem sich in Z-Richtung erstreckenden ersten Gradienten angeordnet. Das Isozentrum 14 beschreibt den Punkt, an dem die XY-Ebene an der Stelle, an der die Z-Komponente B_{Z,Z} des ersten Gradientenfeldes null ist, mit der Symmetrieachse 12 der weiteren Gradientenfelder zusammenfällt. Die symmetrische Anordnung wird dadurch erzielt, dass die mittlere Leiterschleife 11 der drei Leiterschleifen 11, 15 der Diffusionsgradientenspule 10 durch das Isozentrum 14 verläuft und die anderen beiden Leiterschleifen 15 beabstandet davon zu beiden Seiten des Isozentrums 14 angeordnet sind, wobei die drei Leiterschleifen 11, 15 sich jeweils orthogonal zu der Z-Richtung in der XY-Ebene erstrecken. In der XY-Ebene, welche direkt durch das Isozentrum 14 verläuft, findet keine Einkopplung der durch die Gradientenspulen 4, 5, 6 erzeugten Gradientenfelder in die Diffusionsgradientenspule 10 statt. Die Einkopplungen, in die Leiterschleifen 15, welche in beiden Z-Richtungen von dem Isozentrum 14 beabstandet sind, weisen jeweils ein umgekehrtes Vorzeichen auf und heben sich so ganz oder im Wesentlichen ganz auf. Negative Auswirkungen für einen auf der Patientenlagerungseinrichtung 9 gelagerten Patienten, wie beispielsweise eine Vibration und/oder eine Erwärmung der Diffusionsgradientenspule 10, können somit vorteilhaft vermieden werden.

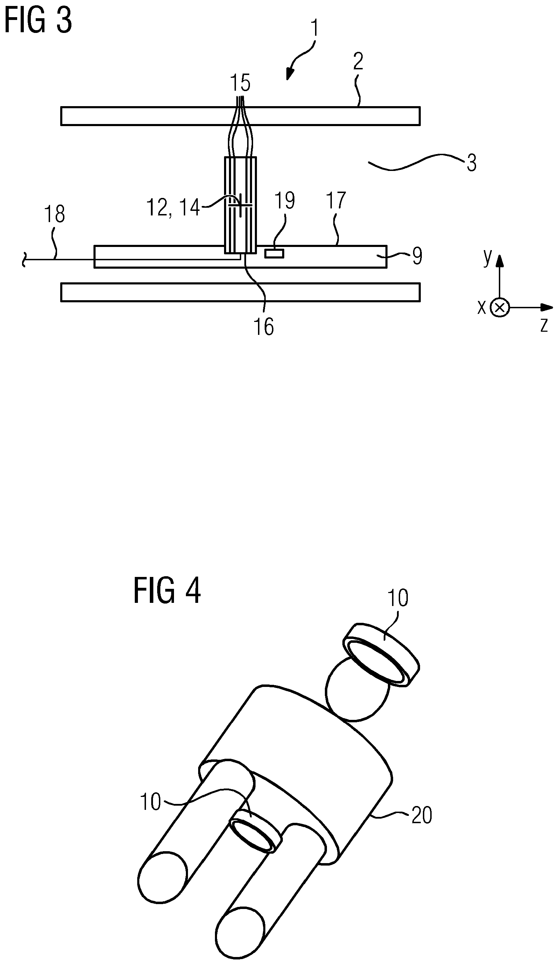

In Fig. 3 ist ein zweites Ausführungsbeispiel einer erfindungsgemäßen Magnetresonanzeinrichtung 1 dargestellt. In diesem Ausführungsbeispiel umfasst die Diffusionsgradientenspule 10 vier Leiterschleifen 15, welche symmetrisch um das Isozentrum 14 angeordnet sind. Die Anordnung der Leiterschleifen 15 in Bezug auf die Symmetrieachse 12 der sich in X-Richtung beziehungsweise Y-Richtung erstreckenden, weiteren Gradienten der weiteren Gradientenfelder ist entsprechend der Anordnung der Leiterschleife 11 zu der Symmetrieachse 12 der Darstellung in Fig. 1. Um eine möglichst genaue Positionierung der Diffusionsgradientenspule 10 in der in Fig. 3 dargestellten Einbauposition zu erreichen, weist die Patientenlagerungseinrichtung 9 eine Aufnahme 16 auf, in welche die Diffusionsgradientenspule 10 formschlüssig und spielfrei eingesetzt ist. Die Halterung 16 ist dabei als Aussparung oder Mulde in einer Patientenlagerungsfläche 17 der Patientenlagerungseinrichtung 19 ausgebildet. Zusätzlich oder alternativ dazu kann die Halterung 16 auch Gegenstücke zu Befestigungsmitteln der Diffusionsgradientenspule 10, wie beispielsweise ein oder mehrere Gewinde zur Aufnahme von Schrauben und/oder eine oder mehrere Mulden zur Aufnahme von Zapfen und/oder Füssen der Diffusionsgradientenspule 10 umfassen. Bei Anordnung der Diffusionsgradientenspule 10 in der Halterung 16 wird erreicht, dass die Diffusionsgradientenspule 10 in Bezug auf das erste und die weiteren Gradientenfelder derart angeordnet ist, dass sowohl von dem ersten Gradientenfeld als auch von den weiteren Gradientenfeldern in der Spule erzeugte Drehmomente und/oder in der Diffusionsgradientenspule erzeugte Ströme sich aufheben oder im Wesentlichen aufheben.

Die Patientenlagerungseinrichtung 9 weist weiterhin eine Leitung 18 auf, welche mit der Diffusionsgradientenspule 10 verbunden ist. Bei der Leitung 18 kann es sich beispielsweise um eine elektrische Leitung und/oder um eine Kühlmittelleitung zur Zuführung eines Kühlmittels, insbesondere eines Öls, an die Diffusionsgradientenspule 10 handeln. Selbstverständlich können mehrere elektrische und/oder fluidische Leitungen vorgesehen und mit der in der Halterung 16 aufgenommenen Diffusionsgradientenspule 10 verbunden sein. Weiterhin umfasst die Patientenlagerungseinrichtung 9 einen der Diffusionsgradientenspule 10 zugeordneten Sensor 19, welcher beispielsweise zur Messung eines von der Diffusionsgradientenspule 10 erzeugten Diffusionsgradientenfeldes und/oder eines Stroms durch die Diffusionsgradientenspule 10 und/oder einer Temperatur der Diffusionsgradientenspule 10 dient. Auch der Sensor 19 kann über hier nicht dargestellte Verbindemittel mit einem außerhalb der Patientenaufnahme 3 angeordneten Auswertemittel und/oder einer Recheneinrichtung oder Ähnlichem verbunden sein.

In Fig. 4 ist schematisch ein Patient 20 dargestellt. An dem Patienten 20 sind zwei Diffusionsgradientenspulen 10 angeordnet. Eine Diffusionsgradientenspule 10 um den Kopf des Patienten angeordnet, wohingegen die andere Diffusionsgradientenspule zwischen den Oberschenkeln des Patienten 20 positioniert ist. Die Diffusionsgradientenspulen 10 können entsprechend ihrer Positionierung beispielsweise für eine Messung am Kopf des Patienten 20 und/oder für eine Messung an der Prostata des Patienten 20 eingesetzt werden. Eine Diffusionsgradientenspule 10 kann dabei abhängig von ihrem Einsatzort beispielsweise eine unterschiedliche Anzahl von Leiterschleifen 11 und/oder Leiterschleifen 15 mit verschiedenen Durchmessern beziehungsweise mit verschiedenen Querschnittsformen aufweisen.

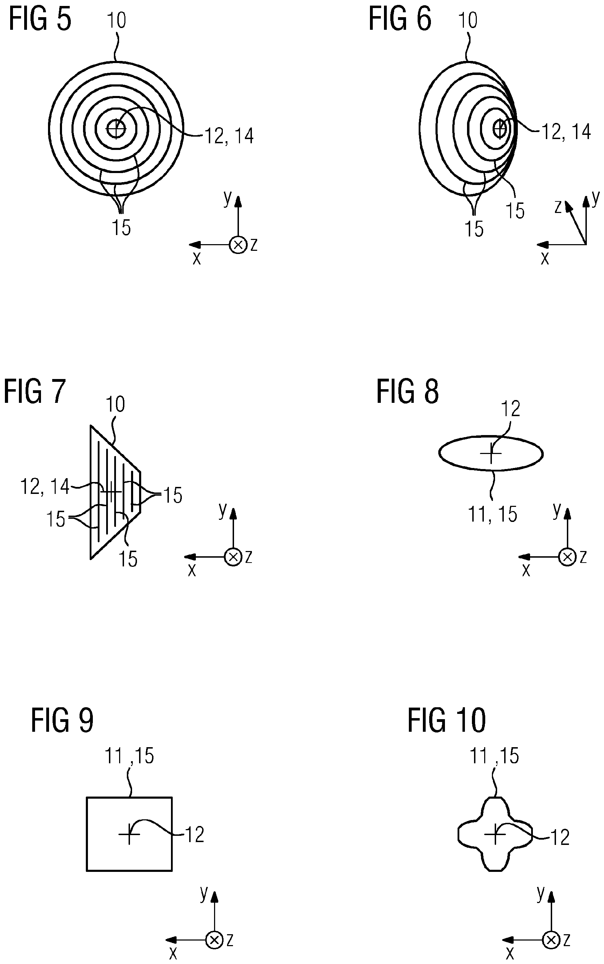

In den Figuren 5 bis 7 sind perspektivische Ansichten eines Ausführungsbeispiels einer Diffusionsgradientenspule 10 dargestellt. Die Diffusionsgradientenspule 10 umfasst mehrere kreisförmige, konzentrisch angeordnete Leiterschleifen 15. In Fig. 5 ist dabei die Anordnung der Diffusionsgradientenspule in der XY-Ebene dargestellt. Die Diffusionsgradientenspule 10 ist dabei derart um das Isozentrum 14 angeordnet, dass sämtliche Mittelpunkte der konzentrischen Kreise in den XY-Ebenen mit der Symmetrieachse 12 zusammenfallen. Zur Verdeutlichung der Anordnung sind weiterhin in Fig. 6 eine Ansicht unter einem Winkel auf die Diffusionsgradientenspule 10 sowie in Fig. 7 die Ansicht der Diffusionsgradientenspule 10 in der YZ-Ebene dargestellt. In Fig. 7 ist beispielsweise zu erkennen, dass die einzelnen Leiterschleifen 15 unterschiedliche Durchmesser aufweisen. Die Anordnung der Diffusionsgradientenspule 10 um das Isozentrum 14 erfolgt beispielsweise derart, dass rechts von dem Isozentrum 14 drei Leiterschleifen 15 mit kleinerem Durchmesser sowie auf er anderen Seite der Isozentren, also in negativer Z-Richtung, zwei Leiterschleifen 15 mit größerem Durchmesser angeordnet sind. Insgesamt ermöglicht es diese Anordnung um das Isozentrum 14, dass aufgrund des ersten Gradientenfeldes erzeugte Drehmomente und/oder durch zeitliche Änderungen dieses ersten Gradientenfeldes in der Diffusionsgradientenspule induzierte Ströme sich ganz oder im Wesentlichen aufheben.

Bei bekanntem Verlauf des ersten Gradientenfeldes sowie der weiteren Gradientenfelder der Magnetresonanzeinrichtung ist es in einem erfindungsgemäßen Verfahren möglich, die Anordnung zueinander und/oder die Querschnittsform der Leiterschleife 11 und/oder der Leiterschleifen 15 der Diffusionsgradientenspule 10 zu bestimmen. Mögliche Verläufe beziehungsweise Geometrien für einzelne Leiterschleifen 11, 15 sind in den Figuren 8 bis 10 dargestellt. Um die Anordnung der einzelnen Leiterschleifen 11, 15 orthogonal zu der Richtung des ersten Gradienten bzw. des Grundmagnetfeldes zu ermöglichen, ist für die Leiterschleifen 11, 15 jeweils vorgesehen, dass diese sich planar in einer Ebene, hier der XY-Ebene, erstrecken. Die notwendigerweise vorhandene Dicke eines Leitungsmaterials zur Bildung der Leiterschleifen 11, 15 sowie gegebenenfalls vorgesehene Verbindungsstücke, welche mehrere Leiterschleifen 11, 15 der Diffusionsgradientenspule zu einer oder mehreren Spulen zusammenschließen, sind in diesen Figuren der Übersichtlichkeit halber nicht dargestellt. Die möglichen Geometrien umfassen eine ovale Geometrie, wie sie in Fig. 8 dargestellt ist, eine quadratische Geometrie, wie sie in Fig. 9 dargestellt ist, sowie eine Geometrie mit polygonförmigem Querschnitt, wie sie in Fig. 10 dargestellt ist.

In den Figuren 8 bis 10 ist weiterhin jeweils die Symmetrieachse 12 der weiteren Gradientenfelder dargestellt, wobei die Leiterschleifen 11 jeweils symmetrisch um diese Symmetrieachse 12 herum angeordnet sind. Durch das erfindungsgemäße Verfahren kann eine Optimierung der Geometrie der Leiterschleife 11 und/oder der Leiterschleifen 15 der Diffusionsgradientenspule erfolgen, wobei als Optimierungskriterium eine Minimierung eines durch eine zeitliche Änderung des wenigstens einen weiteren Gradientenfeldes induzierten Stromes in der Diffusionsgradientenspule 10 und/oder eines durch das wenigstens eine weitere Gradientenfeld erzeugten, auf die Diffusionsgradientenspule wirkenden Drehmoments verwendet werden. Selbstverständlich ist es auch möglich, dass beispielsweise eine Anordnung mehrerer Leiterschleifen 15 in Z-Richtung, wie sie beispielsweise vorangehend in Fig. 7 dargestellt ist, mit Hilfe eines weiteren Optimierungskriteriums hinsichtlich des ersten Gradientenfeldes unter Verwendung des Optimierungsalgorithmus bestimmt wird. Als Randbedingungen der Optimierung können beispielsweise ein minimal erforderlicher Durchmesser einer Leiterschleife 11, 15 oder eine Anzahl von zur Bildung der Diffusionsgradientenspule notwendigen Leiterschleifen 11, 15 verwendet werden. Mit Hilfe des erfindungsgemäßen Verfahrens können somit Diffusionsgradientenspulen 10 erzeugt werden, welche zu einer bestimmten Magnetresonanzeinrichtung 1 beziehungsweise zu einem bestimmten Typ von Magnetresonanzeinrichtungen 1 zugeordnet sind, so dass diese in einfacher Weise in der Patientenaufnahme 3 dieser Magnetresonanzeinrichtung 1 in der Einbauposition angeordnet und insbesondere in einer Aufnahme 16 gehaltert werden können.

Obwohl die Erfindung im Detail durch das bevorzugte Ausführungsbeispiel näher illustriert und beschrieben wurde, so ist die Erfindung nicht durch die offenbarten Beispiele eingeschränkt und andere Variationen können vom Fachmann hieraus abgeleitet werden, ohne den Schutzumfang der Erfindung zu verlassen.

## Patentansprüche

1. Magnetresonanzeinrichtung, umfassend eine Patientenaufnahme (3), wenigstens eine Diffusionsgradientenspule (10), wenigstens einen Magneten zur Erzeugung eines Grundmagnetfeldes und mehrere Gradientenspulen (4, 5, 6) zur Erzeugung von das Grundmagnetfeld überlagernden Gradientenfeldern, wobei sich das Grundmagnetfeld im Wesentlichen in der Patientenaufnahme (3) entlang einer ersten Richtung erstreckt und ein erster Gradient eines erstes Gradientenfeldes in der ersten Richtung sowie wenigstens ein weiterer Gradient eines weiteres Gradientenfeldes in einer weiteren Richtung orthogonal zu der ersten Richtung verläuft, wobei die Diffusionsgradientenspule (10) wenigstens eine in einer Ebene verlaufende Leiterschleife (11, 15) oder mehrere jeweils in parallelen Ebenen verlaufende Leiterschleifen (11, 15) aufweist, **dadurch gekennzeichnet, dass** die Diffusionsgradientenspule (10) derart in der Patientenaufnahme (3) in einer Einbauposition angeordnet ist, dass die Ebene oder die Ebenen der Leiterschleife (11, 15) orthogonal zu dem ersten Gradienten stehen und die Leiterschleife (11, 15) oder die Leiterschleifen (11, 15) in ihren jeweiligen Ebenen in Bezug zu dem wenigstens einen weiteren Gradientenfeld derart verlaufen, dass sich ein durch eine zeitliche Änderung des wenigstens einen weiteren Gradientenfeldes induzierter Strom in der Diffusionsgradientenspule (10) und/oder ein durch das Grundmagnetfeld erzeugtes, auf die Diffusionsgradientenspule (10) wirkendes Drehmoment aufhebt oder im Wesentlichen aufhebt.

2. Magnetresonanzeinrichtung nach Anspruch 1,
**dadurch gekennzeichnet, dass** die Diffusionsgradientenspule (10) in der Einbauposition derart zu dem ersten Gradientenfeld steht, dass sich ein durch das erste Gradientenfeld erzeugtes, auf die Diffusionsgradientenspule (10) wirkendes Drehmoment und/oder ein durch eine zeitliche Änderung des ersten Gradientenfeldes in der Diffusionsgradientenspule (10) induzierter Strom aufhebt oder im Wesentlichen aufhebt.

3. Magnetresonanzeinrichtung nach Anspruch 1 oder 2, **dadurch gekennzeichnet, dass** eine Leiterschleife (11, 15) der Diffusionsgradientenspule (10) und/oder dass alle Leiterschleifen (11, 15) der Diffusionsgradientenspule (10) symmetrisch um ein Isozentrum (14) des ersten Gradientenfeldes angeordnet sind.

4. Magnetresonanzeinrichtung nach einem der vorangehenden Ansprüche, **dadurch gekennzeichnet, dass** die Diffusionsgradientenspule (10) mehrere Leiterschleifen aufweist, wobei die Leiterschleifen (11, 15) konzentrisch und/oder jeweils entlang einer Längsachse der Diffusionsgradientenspule (10) versetzt angeordnet sind und/oder jeweils unterschiedliche Durchmesser aufweisen.

5. Magnetresonanzeinrichtung nach einem der vorangehenden Ansprüche, **dadurch gekennzeichnet, dass** die Leiterschleife (11, 15) oder die Leiterschleifen (11, 15) in ihrer Ebene einen runden, ovalen, rechteckigen oder polygonförmigen Querschnitt aufweisen.

6. Magnetresonanzeinrichtung nach einem der vorangehenden Ansprüche, **dadurch gekennzeichnet, dass** die Diffusionsgradientenspule (10) für eine Messung am Kopf und/oder einer Prostata eines in der Patientenaufnahme (3) positionierten Patienten (20) ausgebildet ist.

7. Magnetresonanzeinrichtung nach einem der vorangehenden Ansprüche, **dadurch gekennzeichnet, dass** die Magnetresonanzeinrichtung (1) wenigstens eine in der Patientenaufnahme (3) und/oder in einer in der Patientenaufnahme (3) angeordneten oder anordenbaren Patientenlagerungseinrichtung (9) ausgebildete Halterung (16) zur entnehmbaren und spielfreien Aufnahme der Diffusionsgradientenspule (10) in der Einbauposition aufweist.

8. Magnetresonanzeinrichtung Anspruch 7, **dadurch gekennzeichnet, dass** Halterung (16) zur formschlüssigen Aufnahme der Diffusionsgradientenspule (10) ausgebildet ist und/oder dass die Halterung (16) zu Befestigungsmitteln der Diffusionsgradientenspule (10) korrespondierende Gegenstücke aufweist.

9. Magnetresonanzeinrichtung nach einem der vorangehenden Ansprüche, **dadurch gekennzeichnet, dass** die Magnetresonanzeinrichtung (1) eine Patientenlagerungseinrichtung (9) umfasst, wobei sich die erste Richtung orthogonal oder parallel zu einer Patientenlagerungsfläche der Patientenlagerungseinrichtung (9) erstreckt und die Diffusionsgradientenspule (10) in oder an der Patientenlagerungseinrichtung (9) angeordnet ist.

10. Magnetresonanzeinrichtung nach Anspruch 9, **dadurch gekennzeichnet, dass** wenigstens ein mit der Diffusionsgradientenspule (10) verbundenes Anschlussmittel, insbesondere eine elektrische Leitung (18) oder ein Kühlmittelschlauch, und/oder wenigstens ein der Diffusionsgradientenspule (10) zugeordneter Sensor (19) in der Patientenlagerungseinrichtung (9) angeordnet ist.

11. Verfahren zur Herstellung einer Diffusionsgradientenspule (10) für eine Magnetresonanzeinrichtung (1) nach einer der vorangehenden Ansprüche, **dadurch gekennzeichnet, dass** der Verlauf und/oder die Querschnittsform der wenigstens einen Leiterschleife (11, 15) der Diffusionsgradientenspule (10) und/oder die Anordnung mehrerer Leiterschleifen (11, 15) der Diffusionsgradientenspule (10) zueinander in Abhängigkeit des von den Gradientenspulen (4, 5, 6) der Magnetresonanzeinrichtung (1) erzeugten wenigstens einen weiteren Gradientenfeldes und der Position der Diffusionsgradientenspule (10) in der Patientenaufnahme (3) unter Verwendung eines Optimierungsalgorithmus bestimmt wird, wobei als Optimierungsbedingung des Optimierungsalgorithmus eine Minimierung eines durch eine zeitliche Änderung des wenigstens einen weiteren Gradientenfeldes induzierten Stromes in der Diffusionsgradientenspule (10) verwendet wird.

12. Verfahren nach Anspruch 11, **dadurch gekennzeichnet, dass** als zusätzliche Optimierungsbedingung eine Minimierung eines durch das erste Gradientenfeld erzeugten, auf die Diffusionsgradientenspule (10) wirkenden Drehmoments und/oder eines durch eine zeitliche Änderung des ersten Gradientenfeldes in der Diffusionsgradientenspule (10) induzierten Stroms verwendet wird.

## Geänderte Patentansprüche

### Geänderte Patentansprüche gemäss Regel 137(2) EPÜ.

1. Magnetresonanzeinrichtung, umfassend eine Patientenaufnahme (3), wenigstens eine Diffusionsgradientenspule (10), wenigstens einen Magneten, welcher zur Erzeugung eines Grundmagnetfeldes ausgebildet ist, und mehrere Gradientenspulen (4, 5, 6), welche zur Erzeugung von das Grundmagnetfeld überlagernden Gradientenfeldern ausgebildet sind, wobei sich das Grundmagnetfeld im Wesentlichen in der Patientenaufnahme (3) entlang einer ersten Richtung erstreckt und ein erster Gradient eines erstes Gradientenfeldes der Gradientenfelder in der ersten Richtung sowie wenigstens ein weiterer Gradient eines weiteres Gradientenfeldes der Gradientenfelder in einer weiteren Richtung orthogonal zu der ersten Richtung verläuft, wobei die Diffusionsgradientenspule (10) wenigstens eine in einer Ebene verlaufende Leiterschleife (11, 15) oder mehrere jeweils in parallelen Ebenen verlaufende Leiterschleifen (11, 15) aufweist, wobei die Diffusionsgradientenspule (10) derart in der Patientenaufnahme (3) in einer Einbauposition angeordnet ist, dass die Ebene oder die Ebenen der Leiterschleife (11, 15) orthogonal zu dem ersten Gradienten stehen und die Leiterschleife (11, 15) oder die Leiterschleifen (11, 15) in ihren jeweiligen Ebenen in Bezug zu dem wenigstens einen weiteren Gradientenfeld derart verlaufen, dass sich ein durch eine zeitliche Änderung des wenigstens einen weiteren Gradientenfeldes induzierter Strom in der Diffusionsgradientenspule (10) aufhebt oder im Wesentlichen aufhebt.

2. Magnetresonanzeinrichtung nach Anspruch 1,
**dadurch gekennzeichnet, dass** die Diffusionsgradientenspule (10) in der Einbauposition derart zu dem ersten Gradientenfeld steht, dass sich ein durch das erste Gradientenfeld erzeugtes, auf die Diffusionsgradientenspule (10) wirkendes Drehmoment und/oder ein durch eine zeitliche Änderung des ersten Gradientenfeldes in der Diffusionsgradientenspule (10) induzierter Strom aufhebt oder im Wesentlichen aufhebt, und/oder die Leiterschleife (11, 15) oder die Leiterschleifen (11, 15) in ihren jeweiligen Ebenen in Bezug zu dem wenigstens einen weiteren Gradientenfeld derart verlaufen, dass sich ein durch ein durch das Grundmagnetfeld erzeugtes, auf die Diffusionsgradientenspule (10) wirkendes Drehmoment aufhebt oder im Wesentlichen aufhebt.

3. Magnetresonanzeinrichtung nach Anspruch 1 oder 2, **dadurch gekennzeichnet, dass** eine Leiterschleife (11, 15) der Diffusionsgradientenspule (10) und/oder dass alle Leiterschleifen (11, 15) der Diffusionsgradientenspule (10) symmetrisch um ein Isozentrum (14) des ersten Gradientenfeldes angeordnet sind.

4. Magnetresonanzeinrichtung nach einem der vorangehenden Ansprüche, **dadurch gekennzeichnet, dass** die Diffusionsgradientenspule (10) mehrere Leiterschleifen aufweist, wobei die Leiterschleifen (11, 15) konzentrisch und/oder jeweils entlang einer Längsachse der Diffusionsgradientenspule (10) versetzt angeordnet sind und/oder jeweils unterschiedliche Durchmesser aufweisen.

5. Magnetresonanzeinrichtung nach einem der vorangehenden Ansprüche, **dadurch gekennzeichnet, dass** die Leiterschleife (11, 15) oder die Leiterschleifen (11, 15) in ihrer Ebene einen runden, ovalen, rechteckigen oder polygonförmigen Querschnitt aufweisen.

6. Magnetresonanzeinrichtung nach einem der vorangehenden Ansprüche, **dadurch gekennzeichnet, dass** die Diffusionsgradientenspule (10) für eine Messung am Kopf und/oder einer Prostata eines in der Patientenaufnahme (3) positionierten Patienten (20) ausgebildet ist.

7. Magnetresonanzeinrichtung nach einem der vorangehenden Ansprüche, **dadurch gekennzeichnet, dass** die Magnetresonanzeinrichtung (1) wenigstens eine in der Patientenaufnahme (3) und/oder in einer in der Patientenaufnahme (3) angeordneten oder anordenbaren Patientenlagerungseinrichtung (9) ausgebildete Halterung (16) zur entnehmbaren und spielfreien Aufnahme der Diffusionsgradientenspule (10) in der Einbauposition aufweist.

8. Magnetresonanzeinrichtung Anspruch 7, **dadurch gekennzeichnet, dass** Halterung (16) zur formschlüssigen Aufnahme der Diffusionsgradientenspule (10) ausgebildet ist und/oder dass die Halterung (16) zu Befestigungsmitteln der Diffusionsgradientenspule (10) korrespondierende Gegenstücke aufweist.

9. Magnetresonanzeinrichtung nach einem der vorangehenden Ansprüche, **dadurch gekennzeichnet, dass** die Magnetresonanzeinrichtung (1) eine Patientenlagerungseinrichtung (9) umfasst, wobei sich die erste Richtung orthogonal oder parallel zu einer Patientenlagerungsfläche der Patientenlagerungseinrichtung (9) erstreckt und die Diffusionsgradientenspule (10) in oder an der Patientenlagerungseinrichtung (9) angeordnet ist.

10. Magnetresonanzeinrichtung nach Anspruch 9, **dadurch gekennzeichnet, dass** wenigstens ein mit der Diffusionsgradientenspule (10) verbundenes Anschlussmittel, insbesondere eine elektrische Leitung (18) oder ein Kühlmittelschlauch, und/oder wenigstens ein der Diffusionsgradientenspule (10) zugeordneter Sensor (19) in der Patientenlagerungseinrichtung (9) angeordnet ist.

11. Verfahren zur Herstellung einer Diffusionsgradientenspule (10) für eine Magnetresonanzeinrichtung (1) nach einer der vorangehenden Ansprüche, **dadurch gekennzeichnet, dass** der Verlauf und/oder die Querschnittsform der wenigstens einen Leiterschleife (11, 15) der Diffusionsgradientenspule (10) und/oder die Anordnung mehrerer Leiterschleifen (11, 15) der Diffusionsgradientenspule (10) zueinander in Abhängigkeit des von den Gradientenspulen (4, 5, 6) der Magnetresonanzeinrichtung (1) erzeugten wenigstens einen weiteren Gradientenfeldes und der Position der Diffusionsgradientenspule (10) in der Patientenaufnahme (3) unter Verwendung eines Optimierungsalgorithmus bestimmt wird, wobei als Optimierungsbedingung des Optimierungsalgorithmus eine Minimierung eines durch eine zeitliche Änderung des wenigstens einen weiteren Gradientenfeldes induzierten Stromes in der Diffusionsgradientenspule (10) verwendet wird.

12. Verfahren nach Anspruch 11, **dadurch gekennzeichnet, dass** als zusätzliche Optimierungsbedingung eine Minimierung eines durch das erste Gradientenfeld erzeugten, auf die Diffusionsgradientenspule (10) wirkenden Drehmoments und/oder eines durch eine zeitliche Änderung des ersten Gradientenfeldes in der Diffusionsgradientenspule (10) induzierten Stroms verwendet wird.
